# EUROPEAN PATENT APPLICATION

(11) **EP 1 283 052 A1**
(43) Date of publication of application: **12.02.2003**
(21) Application number: 01119335.6
(22) Date of filing: 10.08.2001
(51) Int. Cl.: A61K 38/48

(54) **Pharmaceutical composition containing caspase-8 and /or caspase-9 useful for overcoming glucocorticoid- and cancer therapy-induced apoptosis of tumours**

(71) Applicant: Deutsches Krebsforschungszentrum Stiftung des öffentlichen Rechts, 69120 Heidelberg (DE)
(72) Inventor: Herr, Ingrid, 68118 Heidelberg (DE); Debatin, Klaus-Michael, 89075 Ulm (DE)
(74) Representative: Schüssler, Andrea, Dr.

(57) **Abstract**

Described are pharmaceutical compositions containing a caspase-8 and/or caspase-9 protein and/or (a) DNA sequence(s) encoding a caspase-8 and/or caspase-9 protein and a suitable pharmaceutical carrier. Said compositions are useful for restoring apoptosis sensitivity of glucorticoid-induced resistance of carcinoma cells towards cytotoxic therapy.

## Description

The present invention relates to a pharmaceutical composition containing a caspase-8 and/or caspase-9 protein and/or (a) DNA sequence(s) encoding a caspase-8 and/or caspase-9 protein and a suitable pharmaceutical carrier. The present invention also relates to the use of a caspase-8 and/or caspase-9 protein and/or (a) DNA sequence(s) encoding a caspase-8 and/or caspase-9 protein for the preparation of a pharmaceutical composition for restoring apoptosis sensitivity of glucocorticoid-induced resistance of carcinoma cells towards cytotoxic therapy.

Apoptosis signalling converges in the activation of initiator caspases (e.g. procaspase-8, -9) which leads to the proteolytic activation of effector caspases (e.g. caspase-3) that cleave specific substrates. In turn, cross-linking of death receptors by the cognate death ligands and the release of apoptogenic factors from mitochondria elicit the activation of initiator caspases. Chemotherapeutic agents and γ-irradiation facilitate opening of the mitochondrial permeability transition pore and the release of cytochrome c. In the cytoplasm, a holoenzyme complex termed "apoptosome" is formed consisting of Apaf-1, procaspase-9 and cytochrome c to activate downstream effector caspases. The second pathway inducing direct caspase activation originates from death receptor signalling e.g. through CD95 (APO-1/Fas) and its ligand CD95-L (APO-1-L/Fas-L). Binding of CD95-L and similar TNF-family ligands (TNF-α or TRAIL) to their respective death receptors (CD95/APO-1/FAS, TNF-R1, DR4[TRAIL-R1] and DR5[TRAIL-R2], respectively) mediates receptor trimerization and recruitment of adaptor proteins (FADD; TRADD) to the cytoplasmic death domain. FADD recruits procaspase-8 which proteolytically cleaves and thereby activates effector caspases.

Glucocorticoids (GCs) such as the synthetic GC dexamethasone (DEX) are widely prescribed compounds in medical practice today. These steroid hormones are of great value in the treatment of inflammatory disorders and tissue oedema of different origin such as cerebral oedema. GCs possess antipyretic activity, act as antiemetics during chemotherapy and induce apoptotic cell death in many cancer cell types, which has led to their use in the treatment of hematological disorders. Consequently, GCs are given at varying times and at varying doses before, during and after chemotherapy. In addition to their effects on acute toxicity, GCs have a role in the prevention of the late effects of cytotoxic therapy on normal tissues. Thus, GCs could be used to protect normal tissue of cancer patients against the long-term effects of chemotherapy on growth and other late effects as well as to treat acute toxicities. Whilst DEX co-treatment of cancer patients undergoing intensive chemotherapy has important implications for the protection of normal tissue, little is known about molecular mechanisms by which GCs interfere with the apoptotic effects of anti-cancer therapy on the tumour cells themselves. The ability of DEX to induce apoptosis appears to be cell type specific since previous reports show that DEX renders certain hepatoma, glioma, glomerular endothelial cells, and human cervical carcinoma cells resistant to various chemotherapeutic drugs, including methotrexate, cisplatin, vincristine, cytarabine, adriamycin and teniposide. In spite of its clinical importance, the molecular mechanism underlying GC-induced apoptosis is poorly understood and the question why DEX inhibits apoptosis in some cells while directly inducing death in most lymphoid and several other cell lines has not been clarified. Accordingly, it was so far not possible to overcome the problem of GC induced resistance of tumours to cancer therapy.

Thus, the technical problem underlying the present invention is to provide means for overcoming glucocorticoid- and cancer therapy-induced apoptosis resistance of tumours.

The solution to said technical problem is achieved by providing the embodiments characterised in the claims.

It was found by the experiments leading to the present invention that, while dexamethasone induced apoptosis in leukemic T cells, it prevented apoptosis in cervical and lung carcinoma cells following cisplatin, γ-irradiation and ligation of CD95-L, TRAIL- and TNF-α. Dexamethasone induced mitochondrial damage and release of cytochrome c in both cell types but post mitochondrial death signalling in carcinoma cells was blocked by downregulation of caspase-9 expression and activity thereby preventing formation of a functional apoptosome. In contrast, DEX induced caspase-9 activity in leukemic T cells. Similarly, although dexamethasone suppressed cytotoxic therapy-induced expression of death ligands in both cell types caspase-8 activity was prevented in carcinoma cells only. Consequently, caspase-9 activity occurred in leukemic T cells, but not in carcinoma cells following chemotherapy and dexamethasone co-treatment. The suggested cell-type specific mechanism is supported by restorage of apoptosis sensitivity of DEX-treated carcinoma cells towards cytotoxic therapy by liposomal-mediated transfer of caspase-8 and -9 *in vitro* and *in vivo.* These results indicate that the failure of carcinoma cells to undergo cancer therapy-induced apoptosis in the presence of DEX is due to inhibited caspase-8 and -9 signalling. This suggestion is supported by the finding that transfer of the genes as well as the active proteins for caspase-8 and -9 restores the sensitivity of DEX-treated carcinoma cells to cisplatin-induced apoptosis *in vitro* and *in vivo.* In conclusion, high dose glucocorticoids in the therapy of leukemias and lymphomas and as antiemetics or preservatives for normal cells during chemotherapy of solid tumours may render certain tumours resistant or less susceptible to apoptosis following cancer therapy. Thus, transfer of caspase-8 and/or caspase-9 (protein or corresponding DNA sequences) is a powerful therapeutic approach to overcome glucocorticoid- and cancer therapy-induced apoptosis resistance of tumours, i.e. to make the tumours again susceptible to cytotoxic therapy.

Accordingly, the present invention relates to a pharmaceutical composition containing a caspase-8 and/or caspase-9 protein and/or (a) DNA sequence(s) encoding a caspase-8 and/or caspase-9 protein. Preferably, said pharmaceutical composition contains a caspase-8 and caspase-9 protein and/or DNA sequences encoding a caspase-8 and caspase-9 protein.

Sources for the caspase-8 and/or caspase-9 protein and/or (a) DNA sequence(s) encoding a caspase-8 and/or caspase-9 protein are known to the person skilled in the art and, furthermore described in the examples, below.

The terms "caspase-8" and "caspase-9" comprise the naturally occurring proteins as well as derivatives having substantially the same biological activity. These terms also comprise the pro-forms of the active proteins, i.e. procaspase-8 and procaspase-9. The term "derivative" in this context means that the amino acid sequences of these molecules differ from the amino acid sequences of the naturally occurring proteins. The deviations may have been produced by deletion, substitution, insertion or recombination.

The variations as regards the derivatives can be naturally occurring variations, for example amino acid sequences from other organisms, or mutations that can either occur naturally or that have been introduced by specific mutagenesis. Furthermore, the variations can be synthetically produced sequences. The allelic variants can be either naturally occurring variants or synthetically produced variants or variants produced by recombinant DNA processes.

Preferably, the caspase-8 and caspase-9 protein is recombinantly produced by cultivating a host cell transformed with a vector described below under conditions allowing the synthesis of the protein and the protein is subsequently isolated from the cultivated cells and/or the culture medium. Isolation and purification of the recombinantly produced proteins may be carried out by conventional means including preparative chromatography and affinity and immunological separations involving affinity chromatography with monoclonal or polyclonal antibodies.

For recombinant production of caspase-8 and caspase-9 or therapeutic purposes, the DNA sequences encoding caspase-8 and/or caspase-9 are inserted in a recombinant vector, e.g. an expression vector, e.g. a vector suitable for gene therapy. Preferably, the vectors are plasmids, cosmids, viruses, bacteriophages and other vectors usually used in the field of genetic engineering. Vectors suitable for use in the present invention include, but are not limited to the T7-based expression vector for expression in bacteria, the pMSXND expression vector for expression in mammalian cells and baculovirus-derived vectors for expression in insect cells. Preferably, the DNA sequences are operatively linked to the regulatory elements in the recombinant vector that guarantee the transcription and synthesis of an RNA in prokaryotic and/or eukaryotic cells that can be translated. The nucleotide sequence to be transcribed can be operably linked to a promoter like a T7, metallothionein I or polyhedrin promoter.

Preferred recombinant vectors useful for gene therapy are viral vectors, e.g. adenovirus, AAV, herpes virus, vaccinia, or, more preferably, an RNA virus such as a retrovirus or the subgroup lentivirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotid encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, i.e. the carcinoma, the nucleic acids can also be operably linked to a tissue specific promoter and used for gene therapy. For example, the nucleic acid molecule can be ligated to hCMV promoter, MMTV (Mouse mammary tumor virus) promoter, calcium-calmodulin-dependent kinase promotor (for brain-specific expression), prion protein promoter (for brain-specific expression), neuron-specific enolase promoter (for brain-specific expression), LAP promoter (for liver specific expression), rat alpha myosin heavy chain promoter (for heart specific expression), Clara cell 10-Kd protein (CC10) promoter (for lung-specific expression), human keratin 14 (K14) promoter (for expression in epidermis, oral epithelia and esophagus), Tek promotor (for endothelial-specific expression), β-lactoglobulin promoter (for mammary gland-specific expression) or tyrosinase promoter (for melanoycte-specific expression). Other tissue specific promoters are well known to those skilled in the art. Alternatively, the nucleic acids can be linked to an ElA-inducible promoter since many carcinomas but not normal cells express this protein.

For administration, the above described compounds are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperitoneal, subcutaneous, intramuscular, topical or intradermal administration. The route of administration, of course, depends on the nature of the tumour and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the tumour, general health and other drugs being administered concurrently.

The delivery of the above described DNA sequences encoding caspase-8 and/or caspase-9 can be achieved by direct application or, preferably, by using one of the recombinant expression vectors described above or a colloidal dispersion system. These nucleic acids can also be administered directly to the target site, e.g., by ballistic delivery, as a colloidal dispersion system or by catheter to a site in artery. The colloidal dispersion systems which can be used for delivery of the above DNA-sequences include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired DNA sequence. Organ-specific or cell-specific liposomes can be used in order to achieve delivery only to the desired tumour. The targeting of liposomes can be carried out by the person skilled in the art by applying commonly known methods. This targeting includes passive targeting (utilising the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumours via specific cell-surface ligands. The caspase-8 and caspase-9 proteins can also be delivered using the above described systems, preferably by using a liposome.

The present invention also relates to the use of a caspase-8 and/or caspase-9 protein and/or (a) DNA sequence(s) encoding a caspase-8 and/or caspase-9 protein for the preparation of a pharmaceutical composition for restoring apoptosis sensitivity of glucocorticoid-treated carcinoma cells towards cytotoxic therapy. Preferably, the carcinoma is a hepatoma, glioma, lung carcinoma, colon carcinoma, pancreas carcinoma, mamma carcinoma or cervical carcinoma.

### Brief description of the drawings

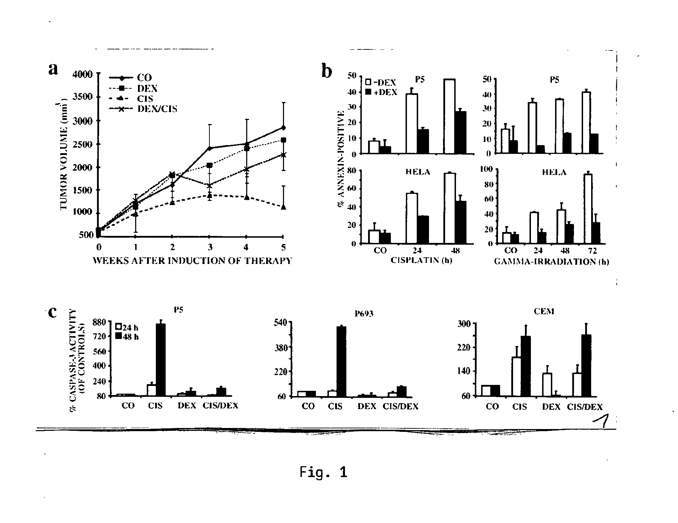

### Figure 1: DEX inhibits therapy-induced carcinoma regression in vitro and in vivo

**(a)** Human epidermoid lung cancer cells (P693) were injected into nude mice. At a tumour volume of 500 mm³ weekly treatment with cisplatin (5mg/kg/week) was started. Cisplatin was injected intraperitoneally in the absence (CIS) or presence (DEX/CIS) of DEX which was added to the drinking water (0.28 mg/L) for the duration of the experiment. The daily rate of DEX consumed was approximately 30 ng/g body weight. As controls, tumor growth of xenografts from mice which received neither cisplatin nor DEX (CO) or from mice which received DEX only (DEX) was determined. The tumour volume in each animal was measured at weekly intervals for a period of 5 weeks. Data are presented as the means +/- SD of 6 to 8 animals.
**(b)** The human cervical carcinoma cell lines HeLa and P5 were γ-irradiated (10 Gy) or treated with cisplatin (2 µg/ml) in the absence (white bars) or presence (black bars) of DEX (1 µM). The percentage of apoptosis was measured in untreated (CO) and stimulated cells by annexin-staining and FACS-analysis at the time points indicated. (c) P5, P693 and human leukemic T cells (CEM) cells were treated with cisplatin (2 µg/ml) in the absence or presence of DEX as indicated. 24 and 48 h following treatment cells were lysed and caspase-3 activity was determined by a fluorescent caspase-3 assay and spectroscopy. The results depicted here are the mean of three separate experiments in duplicates and SD are given.

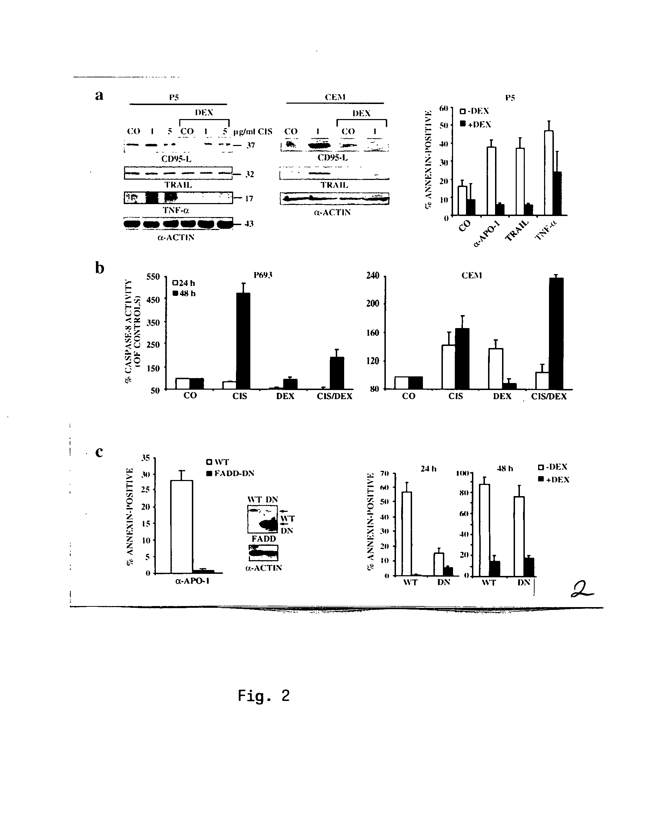

### Figure 2: DEX inhibits expression of death ligands in carcinoma and leukemic T cells but enhances caspase-8 activity in CEM while repressing it in P693 cells

**(a)** Left: P5 an CEM cells were treated with cisplatin (1-5 µg/ml) in the absence or presence of DEX (1 µM) as indicated. Proteins were extracted and expression of CD95-L, TRAIL or TNF-α was detected by Western blot analysis. The size of the proteins in kDa is indicated. Right: HeLa and P5 were treated with CD95 agonistic antibody αAPO-1 (CD95, 1 µg/ml), recombinant TRAIL protein (TRAIL, 100 ng/ml) or recombinant TNF-α protein (TNF-α, 100 ng/ml) in the absence (white bars) or presence (black bars) of DEX (1 µM). 24 h later the percentage of apoptosis was determined by annexin-staining and FACS-analysis in untreated (CO) and stimulated cells at the time points indicated.
**(b)** P693 and CEM cells were treated with cisplatin (2 µg/ml) in the absence or presence of DEX as indicated. 24 and 48 h following treatment cells were lysed and caspase-8 activity was determined by a fluorescent caspase-8 assay and spectroscopy.
**(c)** P5 cells were stable transfected with empty vector (pcDNA3), or pcDNA3 vector encoding dominant negative FADD protein (FADD-DN). Proteins were extracted from both cell lines and expression of wildtype (WT) and FADD-DN (DN) was detected by Western blot. Left: Stable transfected P5 cells were treated with αAPO-1 (1 µg/ml) and 24 h later apoptosis was determined by staining with annexin/propidium iodide and flow-cytometry. Right: stable transfected P5 cells were treated with cisplatin (2 µg/ml) in the absence (white bars) or presence (black bars) of DEX (1 µM). 24 h or 48 h later the percentage of specific apoptosis was determined. The percentage of specific apoptosis was calculated as follows: 100 x [experimental apoptosis (%) - spontaneous apoptosis in the control (%)] / 100% - spontaneous apoptosis in the control (%). The results depicted here have been performed three times with similar outcome and SD are given.

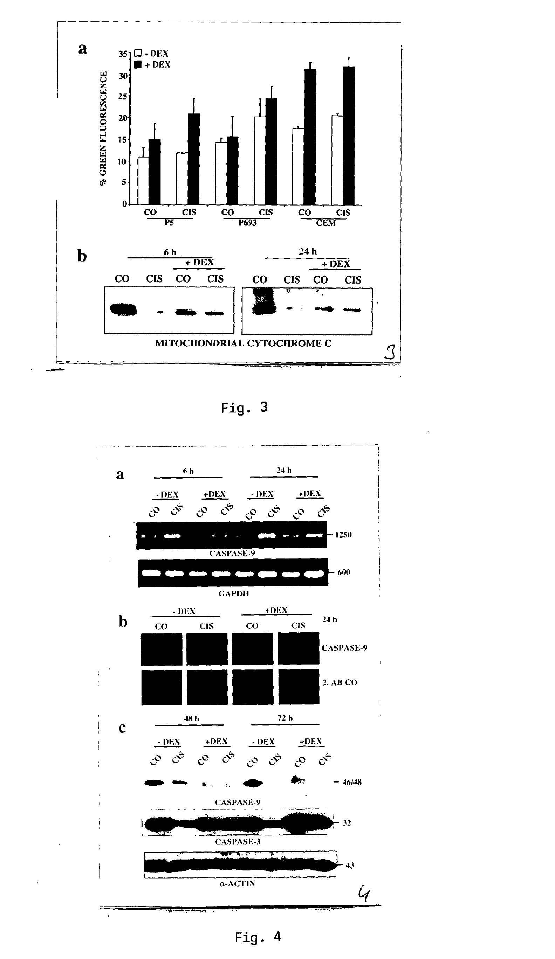

### Figure 3: DEX induces loss of mitochondrial membrane potential and cytochrome c efflux in carcinoma and CEM cells

**(a)** P5, P693 or CEM cells, either untreated (CO) or stimulated with cisplatin (CIS, 2 µg/ml) were incubated in the absence (white bars) or presence (black bars) of DEX (1 µM). 24 h later cells were stained with the cationic dye JC-1 which indicates mitochondrial depolarization by an increase in green fluorescence. The percentage of green-fluorescent cells was determined by flow cytometry.
**(b)** P5 cells were treated as described above. 6 or 24 h later mitochondrial fractions were prepared and assayed by Western blotting using a monoclonal antibody which specifically detects the 15 kDa cytochrome c protein. The results depicted here have been performed three times with similar outcome.

### Figure 4: DEX prevents expression of caspase-9 in carcinoma cells

P5 cells either untreated (CO) or stimulated with cisplatin (CIS, 2 µg/ml) were incubated in the absence or presence of DEX (1 µM).
**(a)** 6 and 24 h later RNA was extracted and assayed by RT-PCR for expression of caspase-9. Expression of the GAPDH gene served as control for equal conditions.
**(b)** 24 h after stimulation expression of caspase-9 proteins was examined by immunofluorescence staining using a rabbit polyclonal caspase-9 antibody and a FITC-coupled secondary antibody. Staining with the secondary antibody alone served as control for unspecific binding.
**(c)** 48 and 72 h after treatment whole cell proteins were extracted and analysed for expression of caspase-9 and caspase-3. The protein sizes in kDa are indicated on the right. Expression of the α-actin protein served as control for equal conditions. The results depicted here have been performed three times with similar outcome.

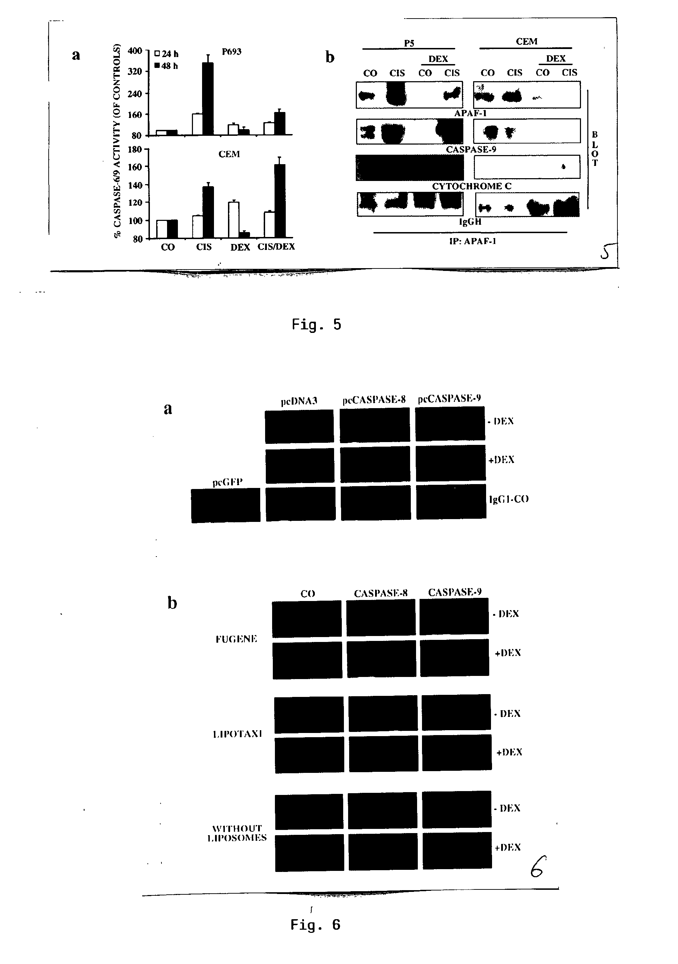

### Figure 5: DEX prevents activation of caspase-9 and formation of the apoptosome in carcinoma cells

**(a)** P693 and CEM cells were treated with cisplatin (2 µg/ml) in the absence or presence of DEX (1 µM) as indicated. 24 and 48 h following treatment cells were lysed and caspase-9 activity was determined using a fluorescent caspase-9 assay and spectroscopy.
**(b)** P5 and CEM cells were treated as described above. 24 h after stimulation Apaf-1 was immunoprecipitated from the cell extracts using a mouse specific for Apaf-1. Aliquotes of the precipitates were separated by three different gels and analysed by Western blotting using rabbit polyclonal Apaf-1, mAb cytochrome c or caspase-9 antibodies. The antibody heavy chain band (IgGH) ensures equal conditions. The results depicted here have been performed three times with similar outcome.

### Figure 6: Liposomal transfer of caspase-8 and -9 proteins and genes overcomes DEX-induced resistance of carcinoma cells in vitro

**(a)** P5 cells were transfected with pcDNA3 empty vector (pcDNA3), or pcDNA3caspase-8 (pcCASPASE-8), -9 (pcCASPASE-9) and pcDNA3GFP (pcGFP) expression vectors using FUGENE liposomes as vehicle. 72 h following transfection apoptosis was detected by staining of the cells with mAb M30 which detects cleaved cytokeratin reflecting caspase activity. Bound antibody was visualised by a FITC-coupled secondary antibody and fluorescence microscopy. A mouse IgG1 antibody (clone DAK-GO1) directed towards *Aspergillus niger* glucose oxidase, an enzyme which is neither present nor inducible in mammalian tissues served as control for specific binding of the primary antibody.
**(b)** P5 cells were transfected with active recombinant caspase-8 and -9 proteins by liposomal mediated protein-transfer using Fugene or Lipotaxi liposomes. The control contained liposomes only (CO). Efficiency of liposomal protein transfer was tested by adding recombinant caspases without liposomes (without liposomes). The amount of apoptosis was determined by staining of the cells with mAb M30 and fluorescence microscopy.

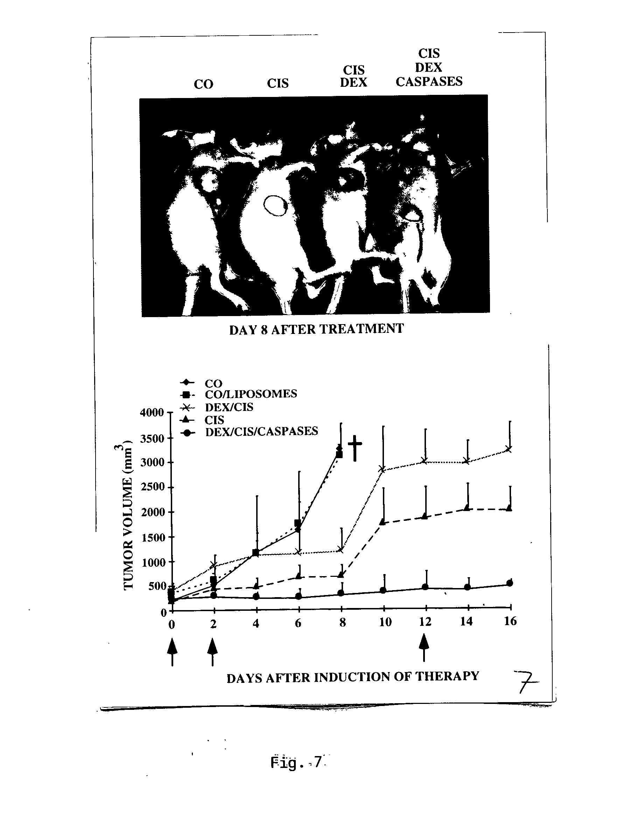

### Figure 7: Liposomal transfer of caspase-8 and -9 proteins and genes overcomes DEX-induced resistance of carcinoma cells in vivo

P5 cells were injected subcutaneously in nude mice. At a tumour volume of 500 mm³ therapy was started (day 0) and repeated at day 2 and 12 as indicated by arrows. A total volume of 100 µl in PBS was injected intratumoural containing liposomes and cisplatin (5mg/kg), or liposomes with cisplatin and a mixture of recombinant caspase-8 and -9 proteins (10 U each) and pcDNA3caspase-8 and pcDNA3caspase-9 expression vectors (10 µg each) in the absence (CIS) or presence of DEX (DEX/CIS), (DEX/CIS/CASPASES) in the drinking water (0.28 mg/L). As controls, tumour growth of xenografts from untreated mice (CO) or from mice which received liposomes and empty pcDNA3 expression vector only (CO/Liposomes) was determined. The tumour volume in each animal was measured every second day for a period of 16 days. At tumour volumes of 3000 mm³ mice were sacrificed as indicated by a cross. Data are presented as the means +/- SD of 6 to 8 animals.

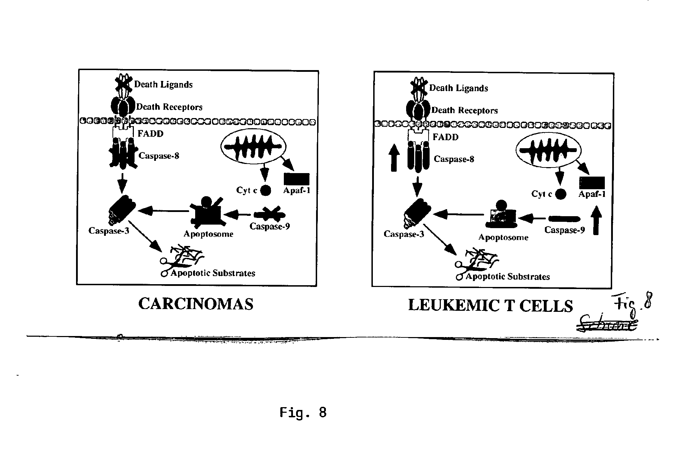

### Figure 8: Schematic representation of DEX-mediated apoptosis regulation in carcinoma cells and leukemic T cells

DEX inhibits basal and therapy-induced expression of the death ligands CD95-L, TRAIL, TNF-α in carcinoma and leukemic T cells. Inhibition of death ligands in carcinoma cells is associated with the failure to activate caspase-8, DEX directly induces activity of caspase-8 in leukemic T cells. DEX provokes mitochondrial damage and the release of cytochrome c in both cell types but inhibits post mitochondrial death signalling in carcinoma cells by preventing caspase-9 expression and activity resulting in a weekly formed and non-functional apoptosome. Combined inhibition of death receptor and mitochondrial death signalling prevents activation of caspase-3 resulting in resistance of carcinoma cells towards cytotoxic therapy-induced apoptosis. Although DEX also slightly inhibits formation of the apoptosome in leukemic T cells, signalling by the apoptosome remains functional due to DEX-enhanced activity of caspase-9 resulting in cleavage of caspase-3 and cell death.

The present invention is explained by the following examples.

### Example 1: Materials and Methods

**(A) Cell culture.** P693 (established directly as a xenograft from a human epidermoid lung carcinoma), P5 (established directly as a xenograft from a human cervix carcinoma) and HeLa (human cervix carcinoma) cell lines (obtained from the Tumorbank Heidelberg) were grown at 37°C in DMEM. CEM (human leukemic T cell line; obtained from the Tumorbank Heidelberg) was grown at 37°C in RPMI. DMEM and RPMI were from Life Technologies Gibco BRL (Karlsruhe, Germany), and were supplemented with 10% fetal bovine serum (Life Technologies).
**(B) Nude Mice and Xenografts.** P693 or P5 cells (10⁶ cells in 200 µl) were injected s.c. into the dorsal surface of 6-10 weeks old NMRI (nu/nu) female mice. The animals were maintained by conventional methods in Makrolon cages at 25°C and 50% humidity. Treatment was started 1 week after transplantation, when the tumor sizes reached about 500 mm³. The mice were given 0.28 mg/L DEX in drinking water, and the amount of water consumed was monitored. Cisplatin was injected intraperitoneally. The tumour volumes were measured using callipers, and calculated using, as an approximation, the formula for volume of a hemi-ellipsoid: π(width² x length)/6. Mice were humanely euthanised at tumour sizes of 3000 mm³.
**(C) Stimulation of cells.** Cisplatin (Sigma, Deisenhofen, Germany) was dissolved in DMSO at a concentration of 10 µg/µl and a 25 mM stock of DEX (Sigma) was prepared in ethanol. Carcinoma cells were pre-treated with DEX three days prior to apoptosis induction. αAPO-1 antibody and recombinant TRAIL protein were prepared as previously described (Dhein et al., Nature 373 (1995), 438-441; Jeremias et al., Eur. J. Imunnol. 28 (1998), 143-152) and human recombinant TNF-α protein was obtained from Sigma. Stock solutions were dissolved in PBS. Final concentrations of the solvents in medium were 0.1% or less. Cells were γ-irradiated in their flasks using a caesium γ-ray source.
**(D) Measurement of apoptosis.** Early apoptotic changes were identified by staining of cells with fluoresceinthiocyanate (FITC)-conjugated annexin V and propidium iodide (Becton Dickinson, Heidelberg, Germany) and analysed by flow cytometry (FACScan, Becton Dickinson) as described (Herr et al., EMBO J. 16 (1997), 6200-6208)
**(E) Immunohistochemistry.** Cells were grown on chamber slides, fixed in 100% methanol and permeabilized in 1% TRITON X 100®. Nonspecific binding was reduced by incubation in 10% Roti®-Immunoblock (Roth, Karlsruhe, Germany). Expression of caspase-9 was detected by a rabbit polyclonal antibody (Santa Cruz, Lexington, USA) and cleavage of cytokeratin was examined by mAB M30 (Roche, Mannheim, Germany). Bound antibodies were detected by FITC conjugated anti-mouse or anti-rabbit IgG (Molecular Probes Europe, Leiden, The Netherlands). Cells were mounted in KAISER's glycerol gelatine (MERCK, Darmstadt, Germany), and examined by fluorescence microscopy. Negative controls were stained without the primary antibody or with mouse IgG1 clone DAK-GO1 (DAKO, Denmark) directed towards *Aspergillus niger* glucose oxidase.
**(F) Western blot analysis.** Protein expression was detected by Western blotting as described (Herr et al., 1997). Mouse monoclonal antibodies were used for detection of CD95-L, TRAIL, FADD and caspase-3 which were obtained from Becton Dickinson. The rabbit polyclonal caspase-9 antibody was from Santa Cruz. A monoclonal antibody against α-ACTIN was from ICN (Eschwege, Germany). Bound antibodies were detected by anti-mouse/horseradish peroxidase conjugates (Santa Cruz) and enhanced chemiluminescence system (Amersham, Braunschweig, Germany). A TNF-α specific goat IgG (R&D Systems, Wiesbaden-Nordenstadt, Germany) in combination with a biotinylated rabbit anti-goat IgG and streptavidin-alkaline phosphatase (Dianova, Hamburg, Germany) was used for detection of TNF-α protein expression. The substrate was developed by incubating in BCIP/NBT-solution (Sigma).
**(G) RT-PCR.** Total RNA was harvested, converted to cDNA and PCR was performed as previously described (Herr et al., Oncogene 19 (2000), 4255-4262). The following primer sequences were used: human caspase-9 (Seol and Billiar, J. Biol. Chem. 274 (1999), 2072-2076): 5'-atggacgaagcggatcggcggc-3' and 5'-ttatgatgttttaaagaaaagttttttc-3' (1250 bp); human GAPDH: 5'-ccacccatggcaaatttctccatggca-3' and 5'-tctagacggcaggtcaggtccacc-3' (600 bp). The cDNA for caspase-8 was obtained from Prof. Dr. Peter Krammer, DKFZ Heidelberg.
**(H) Determination of mitochondrial membrane permeability.** Altered mitochondrial membrane permeability was identified by staining of cells with 10 µg/ml JC-1 according to the instructions of the manufacturer (Molecular Probes, Leiden, The Netherlands). JC-1 is a cationic dye that exhibits potential-dependent accumulation in mitochondria. JC-1 aggregates in intact mitochondria; these aggregates fluorescence red. In cells, in which mitochondrial potential has dropped, JC-1 remains as monomers in the cytoplasm and emits a green fluorescence. Mitochondrial depolarization was detected by FACS-analysis and measuring the percentage of green-fluorescent cells.
**(I) Cytochrome c release assay.** The release of cytochrome c from mitochondria was measured as previously described (Bossy-Wetzel and Green, Meth. Enzymol. 322 (2000), 235-242). In brief, mitochondrial fractions were prepared and analysed by Western blotting using a mouse monoclonal antibody against cytochrome c (Clon 7H8.2C12, Becton Dickinson/Pharmingen).
**(J) Co-immunoprecipitation of apoptosome proteins.** Members of the apoptosome were co-immunoprecipitated as previously described (Beere et al., Nature Cell Biol. 2 (2000), 469-475). In brief, Apaf-1 was immunoprecipitated using a monoclonal antibody (Becton Dickinson/Pharmingen, Heidelberg, Germany). The immunoblots were stained with rabbit polyclonal antibodies Apaf-1 (a gift from Dr. Xiaodong Wang), caspase-9 (Santa Cruz, Lexington, USA) or mAB cytochrome c (Becton Dickinson/Pharmingen, Heidelberg, Germany).
**(K) Detection of caspase activity.** Cell lysates were incubated with specific substrates for caspase-3, -8 and -9 according to the instructions of the manufacturer (Clontech, Heidelberg, Germany) and caspase activity was analysed in a Victor fluorometer (Wallac, Freiburg, Germany).
**(L) cDNA constructs in the mammalian pcDNA3 expression vector.** The cDNA for caspase-9 inserted XbaI in pBluescript SK (+/-) was kindly provided by Dr. D. W. Nicholson. The caspase-9 cDNA was subcloned into pcDNA3 using the restrictions sites NotI and EcoRI. pcDNA3 expression vectors for dominant negative FADD and caspase-8 were kindly provided by Dr. V. Dixit and Dr. P. H. Krammer, respectively. pcDNA3-GFP was from Clontech (Heidelberg, Germany).
**(M) Stable transfectants.** P5 cells were washed in PBS, resuspended at 5 x 10⁷ cells/200 µl PBS and transfected with 50 µg of a pcDNA3-FADD-dominant negative expression construct or empty pcDNA3 vector by electroporation (975 µF, 220 V). Resistant cells were selected in medium containing 1 mg/ml G418 (Calbiochem, Bad Soden, Germany) over a period of six weeks.
**(N) Liposomal-mediated transfer of caspase-8 and -9.** P5 cells were grown on chamber slides and recombinant human caspase-8 or -9 proteins (ALEXIS/BioVision, Gruenberg, Germany) were added at a concentration of 1 U/150 µl medium using Fugene (Roche, Mannheim, Germany) or LipoTaxi liposomes (Stratagene, La Jolla, USA) liposomal transfection systems according to the instructions of the manufacturer. pcDNA3-caspase-8, pcDNA3-capase-9 or pcDNA3-GFP were added at a concentration of 1 µg/150 µl medium using Fugene liposomes. A mixture of recombinant caspase-8 and -9 proteins (10 U each) and pcDNA3-caspase-8 and -9 vectors (10 µg each) was injected in P5 xenografts using Fugene liposomes as vehicle.

### Example 2: DEX prevents cisplatin-induced apoptosis in human carcinoma cells in vitro and in vivo but enhances cell death in leukemic T cells

To assess the effect of DEX in combination with cytotoxic tumour therapy on human carcinoma cells, patient-derived P693 lung carcinoma xenografts were grown in nude mice. At a tumour size of 500 mm³, cisplatin was injected intraperitoneally weekly over a period of five weeks in the absence or presence of DEX which was added to the drinking water. Weekly measurement of the tumour volume revealed profound reduction of tumour growth in cisplatin-treated mice which was diminished by DEX since the tumours grow faster in combination treatment (Fig. 1a). Tumour growth of untreated control mice or of mice receiving DEX only increased continuously.

To investigate whether this inhibitory effect of DEX is restricted to P693 lung carcinoma cells in addition P5 and HeLa cervix carcinoma cells were examined in *vitro.* Cells were treated with cisplatin or γ-irradiated in the presence or absence of DEX. Apoptosis was determined by staining of the cells with annexin-V/FITC and flow cytometry (Fig. 1b). Apoptosis was detectable 24 h following treatment and increased during the next 48 and 72 h. Combined DEX treatment strongly suppressed the level of apoptosis induced by cisplatin or γ-irradiation. Furthermore, DEX co-treated cells remained viable and proliferated as observed by microscopy. The above results were confirmed by the measurement of caspase-3 activity using a fluorometric assay. Caspase-3 activity was induced 48 h following cisplatin in P5 and P693 cells, whereas CEM cells responded earlier at 24 h with increased activity at 48 h (Fig. 1c). In the presence of DEX caspase-3 activity was completely blocked in carcinoma cells whereas in CEM cells induced activity was not affected and DEX alone mediates an induction of caspase-3. Thus, DEX neutralises cancer therapy-induced apoptosis in several carcinoma cells *in vitro* and *in vivo* whereas inducing apoptosis in leukemic T cells.

### Example 3: Suppression of cisplatin-induced death receptor signaling by DEX in human carcinoma cells but not in lymphoid T cells

Mechanistical data on pro- or anti-apoptotic effects of GCs on cytotoxic therapy are sparse and conflicting. Therefore it was examined whether DEX may interfere with death receptor signalling. To measure expression of death ligands (DILs) we treated P5 and CEM cells with cisplatin in presence or absence of DEX. Western blot analysis revealed enhanced expression of DILs such as CD95-L, TRAIL and TNF-α after stimulation with cisplatin alone in both cell types with a constitutive high expression of TRAIL in P5 cells (Fig. 2a). DEX co-treatment downregulated the expression of basal and induced expression of each DIL in both cell lines. Whereas this result was expected for the apoptosis-resistant carcinoma cells it was unexpected for the apoptosis-sensitive leukemic T cells. To examine whether DILs are able to induce apoptosis in carcinoma cells and therefore may contribute to cisplatin-induced apoptosis the death receptors of P5 cells were stimulated by exogenously added agonistic αAPO-1 receptor antibody (αAPO-1), recombinant TRAIL or TNF-α proteins. 24 h later apoptosis was enhanced by any DIL suggesting that cisplatin-induced expression of DILs contributes to chemotherapy-mediated apoptosis. DEX co-treatment strongly repressed the induction of death receptor-mediated apoptosis. In contrast, and despite inhibition of DILs, death receptor-mediated apoptosis was superinduced by co-treatment with DEX in CEM cells (data not shown). Thus, DEX may exert its effects by targeting elements upstream and downstream of death receptors.

To assess the influence of DEX downstream of death receptors activity of caspase-8 was examined by a specific fluorometric assay. Activity of caspase-8 was induced 48 h following treatment with cisplatin in P693 cells, whereas CEM cells responded earlier at 24 h with increased activity at 48 h (Fig. 2b) The increase in caspase-8 activity was completely blocked in the presence of DEX in P693 but not in CEM cells, where activity was superinduced at 48 h. The mechanism by which DEX directly activates caspase-8 while concomitantly suppressing DIL expression in leukemic T cells is unknown.

Next it was investigated whether DEX may already influence FADD signalling as the main inducer of caspase-8. To mimic the action of DEX the signal transduction pathway downstream of death receptors was blocked by stable transfecting FADD-dominant negative (FADD-DN) to P5 cells. Western blot analysis ensured expression of FADD-DN which blocked death receptor signalling as tested by triggering of CD95 with the agonistic antibody αAPO-1 (Fig. 2c left). In line with this finding, FADD-DN inhibited cisplatin-induced apoptosis 24 h after treatment (Fig. 2c right). However, at 48 h, when cisplatin-induced apoptosis reached levels of about 80%, no further inhibitory effect of FADD-DN was detectable suggesting that inhibition of FADD is not the main underlying mechanism for the inhibitory effect of DEX which still diminished cisplatin-induced apoptosis at 48 h in wildtype and FADD-DN expressing cells. Thus, blockade of DIL expression and consecutive death signalling by FADD and caspase-8 may be one but not the only effector in DEX-mediated suppression of apoptosis in carcinoma cells. In contrast, circumventing blocked expression of DILs by direct activation of caspase-8 may contribute to DEX-induced apoptosis in leukemic T cells.

### Example 4: DEX prevents signalling by the caspase-9/apoptosome complex in carcinoma cells despite cytochrome c efflux from mitochondria

Mitochondria represent another apoptosis pathway which may be affected by DEX. P5, P693 and CEM cells were treated with cisplatin in the presence or absence of DEX. Mitochondrial function was examined by staining of the cells with JC-1. The percentage of green fluorescent cells corresponding to the degree of mitochondrial damage was analysed by flow cytometry. 24 h following treatment DEX increased the percentage of green fluorescence which was further enhanced by co-stimulation with cisplatin (Fig. 3a). DEX alone or in combination with cisplatin induced efflux of cytochrome c from mitochondria, which was first detectable at 6 h and completed at 24 h as shown for P5 cells, (Fig. 3b). Thus, mitochondrial damage and release of cytochrome c may be involved in DEX-induced apoptosis of leukemic T cells. However, since DEX provokes damage of the mitochondrial membrane potential and release of cytochrome c also in carcinoma cells without inducing apoptosis, DEX may block signalling downstream of cytochrome c in these cells.

To examine the post-mitochondrial effects of DEX on carcinoma cells, P5 cells were treated with cisplatin in the presence or absence of DEX and examined the expression of caspase-9 by RT-PCR (Fig. 4a). Cisplatin strongly enhanced caspase-9 mRNA expression 6 and 24 h following stimulation whereas DEX co-treatment downregulated basal and upregulated expression. Downregulation of caspase-9 expression by DEX was also detectable at the protein level as examined by immunohistochemistry 24 h after stimulation (Fig. 4b) and by Western blot analysis 48 and 72 h following treatment (Fig. 4c). However, at 48 and 72 h not only DEX but also cisplatin reduced the protein levels of procaspase-9. This is most likely due to cisplatin-mediated cleavage of procaspase-9 to its active form which is not detected by the used antibody. This conclusion is supported by the activation pattern of caspase-9 as detected by a fluorometric assay and spectroscopy (Fig. 5a). Also, procaspase-3, the target of active caspase-9, is cleaved 48 and 72 h following cisplatin but remains in its proform in the presence of DEX (Fig. 4c, 2b). Compared to carcinoma cells, expression of caspase-9 was very low in leukemic T cells and neither induced by cisplatin nor inhibited by DEX (data not shown, compare also Fig. 5a). To analyse the influence of DEX on activity of caspase-9 a fluorometric assay specific for caspase-9 and -6 was used and the substrate turnover was detected by spectroscopy. Caspase-6/9 activity was induced 24 h following cisplatin in P693 and CEM cells with increasing activity at 48 h (Fig. 5a). In the presence of DEX caspase-6/9 activity was completely blocked in carcinoma cells but even more induced in CEM cells. Whereas DEX alone had no influence on caspase-6/9 activity in carcinoma cells, it increased caspase-6/9 activity at 24 and 48 h in CEM cells. Repression of caspase-9 expression and activity in carcinoma cells may result in a non-functional apoptosome. To examine whether DEX may influence apoptosome assembly in carcinoma cells, cells were stimulated with cisplatin alone or in combination with DEX and immunoprecipitated endogenous Apaf-1. Aliquots of the precipitates were analysed by Western blot analysis. Basal formation of the apoptosome was visible in P5 and CEM cells in comparable amounts since caspase-9 and cytochrome c were co-immunoprecipitated with APAF-1 (Fig. 5b). In the presence of cisplatin, strong induction in the amounts of APAF-1, caspase-9 and cytochrome c were visible in carcinoma cells but not in leukemic T cells. In the presence of DEX the amounts of immunoprecipitated apoptosome members were strongly inhibited in both cell lines. Thus, DEX prevents cytochrome c signalling by inhibition of caspase-9 expression and activity resulting in a weekly formed and non-functional apoptosome in carcinoma cells. In contrast, although DEX inhibits formation of the apoptosome in CEM cells this may have no influence on apoptosis since activity of caspase-9 is induced and the formation of the apoptosome is per se low in leukemic T cells.

### Example 5: Transfection of caspase-8 and -9 restores apoptosis sensitivity of DEX treated carcinoma cells in vitro and in vivo

Since inhibition of caspase-8 and -9 signalling by DEX may be the main underlying mechanism for the resistance of carcinoma cells to cytotoxic therapy, it was examined whether transfection of caspase-8 and -9 would restore apoptosis sensitivity. P5 cells were transiently transfected with pcDNA3 expression vectors for caspase-8, and -9 or empty vector in the control using Fugene liposomes. A pcDNA3-GFP expression construct served as control for the efficiency of transfection. 72 h after transfection cleavage of cytokeratin reflecting caspase-activity and apoptosis was detectable by immunofluorescence staining. Transfer of each caspase resulted in strong and comparable amounts of cytokeratin cleavage in the absence of DEX (Fig. 6a). However, DEX inhibited vector-mediated expression of caspase-9 whereas overexpressed caspase-8 was not affected. No specific staining was observed in controls, which were incubated with the FITC-coupled secondary antibody only. In contrast, cells transfected with the GFP expression plasmid exhibited strong green fluorescence ensuring a high transfection efficiency. Since DEX also inhibited the expression of transfected caspase-9 active recombinant caspase-8 and -9 proteins were transfected to P5 cells using Fugene or Lipotaxi liposomes. 24 h after transfection cleavage of cytokeratin was detectable by immunofluorescence staining. Transfer of each caspase resulted in strong and comparable amounts of cytokeratin cleavage in the presence or absence of DEX (Fig. 6b). In contrast, no cleaved cytokeratin was observed in controls, to which caspases were transferred in the absence of liposomes suggesting that transfection was dependent on the liposome vesicles. No difference in transfection efficiency using Fugene or Lipotaxi liposomes was detectable.

To test whether the transfer of caspase-8 and -9 would also overcome DEX-induced resistance of carcinoma cells to cytotoxic therapy *in vivo,* P5 cells were injected subcutaneously into nude mice. At a tumour volume of 500 mm³ therapy was started. A mixture of liposomes, caspase-8 and -9 proteins, caspase-8 and -9 pcDNA3 expression vectors and cisplatin were injected intratumoural in the presence or absence of DEX which was added to the drinking water. Injections were repeated two and 12 days later. Whereas the tumour volume of untreated control mice or mice receiving liposomes and empty pcDNA3 expression vector only increased continueously, tumour growth of cisplatin-treated mice was initially reduced (Fig. 7). However, at day 10 after treatment the cisplatin-treated tumours developed resistance and started to grow. In contrast, tumours transfected with the caspase-cocktail did not increase even in the presence of DEX which strongly enhanced growth of cisplatin-treated xenografts. Furthermore, one out of 8 tumours from the caspase-group totally disappeared.

## Claims

1. A pharmaceutical composition containing a caspase-8 and/or caspase-9 protein and/or (a) DNA sequence(s) encoding a caspase-8 and/or caspase-9 protein.

2. The pharmaceutical composition of claim 1, wherein the caspase-8 and/or caspase-9 protein and/or (a) DNA sequence(s) encoding a caspase-8 and/or caspase-9 protein are included in liposomes.

3. Use of a caspase-8 and/or caspase-9 protein and/or (a) DNA sequence(s) encoding a caspase-8 and/or caspase-9 protein for the preparation of a pharmaceutical composition for restoring apoptosis sensitivity of glucocorticoid-treated carcinoma cells towards cytotoxic therapy.

4. Use according to claim 1, wherein the carcinoma is a hepatoma, glioma, lung carcinoma, colon carcinoma, pancreas carcinoma, mamma carcinoma or cervical carcinoma.
